(19) Europäisches Patentamt / European Patent Office / Office européen des brevets

(11) **EP 3 275 394 B1**

(12) **EUROPEAN PATENT SPECIFICATION**

(45) Date of publication and mention
of the grant of the patent:
**27.03.2019 Bulletin 2019/13**

(21) Application number: **16001643.2**

(22) Date of filing: **26.07.2016**

(51) Int Cl.:
***A61B 90/50*** *(2016.01)*

(54) **SUPPORTING ARM SYSTEM**

TRAGARMSYSTEM

SYSTÈME DE BRAS DE SUPPORT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**31.01.2018 Bulletin 2018/05**

(73) Proprietor: **Ondal Medical Systems GmbH
36088 Hünfeld (DE)**

(72) Inventor: **Stroop, Nicolas
33602 Bielefeld (DE)**

(74) Representative: **Graf von Stosch, Andreas et al
Graf von Stosch
Patentanwaltsgesellschaft mbH
Prinzregentenstraße 22
80538 München (DE)**

(56) References cited:
**US-A1- 2007 129 634     US-A1- 2011 303 805
US-A1- 2012 293 937     US-A1- 2014 246 552
US-B1- 7 677 540**

**Description**

Technical Field

The present invention relates to a supporting arm system for supporting technical equipment, especially medical equipment according to claim 1. The supporting arm system comprises a first base element configured to be mounted to a stationary element, a second base element for carrying a technical device, especially a medical device, a supporting arm pivotally mounted to the first base element at a first pivot axis and pivotally mounted to the second base element at a second pivot axis; the supporting arm comprising a first supporting arm member formed as a load arm and a second supporting arm member for stabilizing a position of the load arm, and tensioning means connected to the second member for exerting a tension force on the second supporting arm member to stabilize the supporting arm in a given position. Further, the present invention also relates to a stand for supporting technical equipment, especially medical equipment in a hospital or other medical environment according to the respective independent claim. Also, the present invention also relates to a ceiling mount for supporting technical equipment, especially medical equipment in a hospital or other medical environment according to the respective independent claim.

More particularly, the present invention relates especially to a supporting arm system with individual features of claim 1, to a stand for supporting technical equipment, especially medical equipment in a hospital or other medical environment, with individual features of the respective independent claim and to a ceiling mount for supporting technical equipment, especially medical equipment in a hospital or other medical environment, with individual features of the respective independent claim.

Supporting arm systems for supporting technical equipment, especially medical equipment in a hospital or other medical environment, comprise one or more supporting arms which are at one end pivotably attached to a first base element, such as a supporting or mounting element, ceiling mount, head piece or other, and which are at another end pivotably attached to a second base element, such as a connector, a mounting element, a second head piece or a carrier for a medical device, monitor or other. By means of the pivotable attachment the height above a ground or floor of the second element is adjustable. In addition to the pivotable attachment, the one or more supporting arms are swivel mounted such that the supporting arm may swivel around an axis that is essentially parallel to a gravitational force vector. Accordingly, by means of the supporting arm system one or more second elements like height adjustable carriers for carrying medical devices can be moved and positioned in X, Y and Z direction of a coordinate system, e.g. in an operating room, an intensive care room or in a medical examination room. Medical devices include but are not limited to e.g. medical-electrical terminals, (diagnostic) monitors, lighting facilities etc.

Background of the Invention

In height adjustable supporting arm systems compensation mechanisms are provided which allows for a weight compensation to be carried out in the event of changes in the pivot position in order that the supporting arm remains reliably stationary.

US 2008/0111042 A1 discloses a carrying device for medical devices that has a height-adjustable bracket designed in the form of a parallelogram cranking mechanism with a self-locking pneumatic spring and a further pneumatic spring. A supply unit for medical devices is arranged at one end of the bracket. A handle with an electric release element is arranged at the supply unit. A valve, which releases the blocking function, is opened within the pneumatic spring during the actuation of the release element.

A disadvantage of this cranking mechanism is the number of bearings required for mounting the legs of the parallelogram cranking mechanism to the head elements and for mounting the springs to the legs and the head elements, which makes the device costly.

DE 3312137 A1 discloses a ceiling stand consisting of a ceiling tube which can rotate and is fitted to the room ceiling, and a parallelogram arm, which can pivot and projects out of said ceiling tube, on whose free end an apparatus support is located, with the apparatus which is to be held. Arranged in the ceiling tube is a compensating device which is formed from helical springs and allows weight compensation to be carried out in the event of load changes, in order that the parallelogram arm remains reliably stationary, without any special locking, in any position once it is selected.

US 2007/0187562 A1 discloses a support, which may be a ceiling support, and which is designed to receive an operation microscope, particularly a medical operation microscope. This support has a support column, movable around a rotation axis, with a first carrier arm and a second carrier arm which, with respect to the first carrier arm, can be moved around a first pivot axis and a second pivot axis. A cable connection is provided for producing a restoring force for the second carrier arm, and couples the second carrier and to a counterweight for producing a restoring force around the second pivot axis. The counterweight is arranged in the region of the support column, to minimize inertial action accompanying a movement of the support around the rotation axis. A freewheel rotary joint is provided in the cable connection to suppress a feedback to the counterweight of a movement of the second carrier arm around the first pivot axis.

The disadvantage of this support is that the pivotable counterweight needs a pivoting space outside of the device and that the counterweight adds additional weight to the device.

Summary of the Invention

Accordingly, it is an object of the present invention to provide an improved supporting arm system and a stand for supporting technical equipment including such an improved supporting arm system.

According to one aspect, the second supporting arm member is formed as a bendable tension intermediary which is fixedly connected at one end portion to a transmission member of the tensioning means and which is fixedly connected at its respective other end portion to a first curved guide member that is non-rotatably arranged on the first pivot axis with the bendable tension intermediary being guided around a part of the circumference of the first curved guide member, and wherein a second curved guide member is non-rotatably arranged on the second pivot axis with the bendable tension intermediary being slip-free guided around a part of the circumference of the second curved guide member. With this supporting arm configuration the supporting arm system can be simplified in that the number of necessary pivot axes can be reduced to a minimum of two. Further, due to the generally reduced number of parts, assembly during manufacturing is simplified. Slip-free guidance between the bendable tension intermediary and the second curved guide member may especially be realized by one of a form fit, force fit or frictional engagement.

In a preferred embodiment of the invention, the tensioning means is fixedly mounted at the load arm. Accordingly, the tensioning means is functionally located between the load arm and the bendable tension intermediary such that the tension force of the tensioning means keeps the load arm in equilibrium in each of its possible pivot positions.

In a further preferred embodiment of the invention, the second curved guide member defines a first section of the bendable tension intermediary extending between the tension means and the second curved guide member, and the second curved guide member defines a second section of the bendable tension intermediary extending between the second curved guide member and the first curved guide member, and wherein the first section and the second section run in parallel to each other. With this geometry, a slim outer contour of the supporting arm may be realized.

In a preferred embodiment of the invention, a longitudinal axis of the load arm intersects the first and second pivot axis and runs in parallel with the first section and the second section of the bendable tension intermediary. With such geometry, the momentum to be compensated by the tensioning means is only depending on the load at the second base element, from the axial distance of the first and second curved guide members along the longitudinal axis and from the pivot angle of the load arm.

In a further preferable embodiment of the invention, the bendable tension intermediary is formed as one of a chain, belt, rope or wire rope. A bendable tension intermediary of such type is reliable, durable and ensures low maintenance.

Preferably, the first and second curved guide members are formed as one of gearwheels, belt wheels or pulleys. The first and second curved guide members of such type cooperates with the corresponding bendable tension intermediary of the chain, belt, rope or wire rope type to ensure optimal cooperation between the bendable tension intermediary and the first and second curved guide members.

In a further advantageous embodiment of the invention, the tensioning means comprises at least one spring element from the group consisting of tension spring, pressure spring and pneumatic spring. The tension spring and pressure spring is mechanically simple and of relative low cost. The gas spring on the other hand has a relative linear spring characteristic when compared to a mechanical spring. With all such types of spring elements the counter-momentum to the momentum at the pivot axis of the first base element may be realized in a simple way. Preload of the spring element, the diameter of the first and second curved guide member and spring constant of the spring element control the counter-momentum, whereby the counter-momentum may easily be adjusted for a given load range by choosing the optimal pretension.

In an alternative embodiment of the invention, the tensioning means comprises a motorized actuator which enables an automated lifting/lowering of the second base member relative to the first base member of the supporting arm system. Advantageously the motorized actuator is electrically controlled by a (remote) control unit through which a user may exercise control.

Advantageously, the motorized actuator includes an electrically controllable motor which is coupled to the transmission member that is connected to the bendable tension intermediary. Such electrically controllable motor constitutes an inexpensive solution of a remotely controllable automated lifting/lowering drive for the supporting arm system.

Preferably, the motor cooperates with a worm gear that is coupled to the transmission member. The drive transmission via the worm gear is self-locking such that when the motor is de-energized, the supporting arm system will maintain its pivot position without any additional means for holding the second base member in the actual position.

Preferably, the first and second curved guide members have identical diameters and circumferences. Due to their identical diameters, the first and second curved guide members maintain their respective rotary positions with respect to their base elements when the supporting arm is pivoted and the second base member is lifted or lowered.

In a further preferred embodiment of the invention, the first and second curved guide members are elliptical having a first smaller diameter and a second larger diameter and wherein both diameters are perpendicular to each other. With such elliptical configuration of the gearwheels the non-linear momentum at the first pivot axis can be approximated further to the counter momentum of the tensioning means / pressure spring such that the equilibrium of the supporting arm is optimized over the entire range of pivotal movement.

According to a further aspect, the present invention provides a stand for supporting technical equipment, especially medical equipment in a hospital or other medical environment, comprising one or more supporting arm systems according to one or more of the preceding claims. The stand including the supporting arm system realizes all the advantages, as described above.

According to a still further aspect, the present invention provides ceiling mount for supporting technical equipment, especially medical equipment in a hospital or other medical environment, comprising one or more supporting arm systems according to one or more of the preceding claims. The ceiling mounted support including the supporting arm system realizes all the advantages, as described above.

The ceiling mount may also be mounted to a side wall of a room.

Brief Description of the Drawings

The above and further features and advantages of the invention will become more readily apparent from the following detailed description of preferred embodiments of the invention with reference to the accompanying drawings, in which like reference characters identify like features, and in which:

Fig. 1 is a side view of a supporting arm system according to an embodiment of the invention with a supporting arm in a first position with respect to first and second base elements with the arm system being mounted on a movable stand and with a medical device mounted to the supporting arm system;

Fig. 2 is a cut side view of the supporting arm system of Fig. 1;

Fig. 3 is a cut side view of the supporting arm system according to Figures 1 and 2 displaying a different position of the supporting arm compared to Figures 1 and 2;

Fig. 4 is a cut side view of a supporting arm system according to a further embodiment of the invention with a supporting arm in a first position with respect to first and second base elements;

Fig. 5 is a cut side view of a supporting arm system according to a further embodiment of the invention with a supporting arm in a first position with respect to first and second base elements;

Fig. 6 is a side view of a supporting arm system according to a further embodiment of the invention with a supporting arm in a first position with respect to first and second base elements with the arm system being mounted to a ceiling and with a medical device mounted to the supporting arm system;

Fig. 7 is a side view of a supporting arm system according to a further embodiment of the invention with a supporting arm in a first position with respect to first and second base elements with the arm system being mounted on a movable stand and with a medical device mounted to the supporting arm system;

Fig. 8 is a side view of a supporting arm system according to a further embodiment of the invention with a supporting arm in a first position with respect to first and second base elements with the arm system being mounted on a movable stand and with a medical device mounted to the supporting arm system.

Detailed Description of the Preferred Embodiments

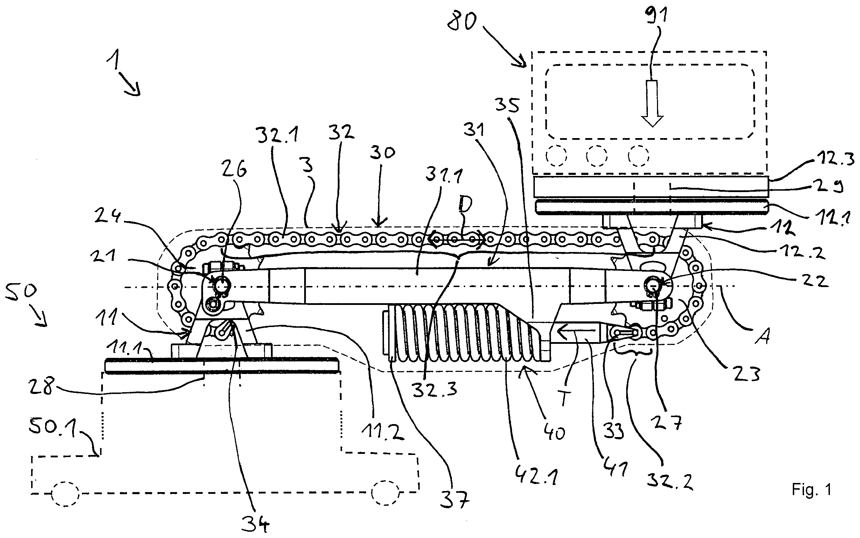

Figures 1 to 3 show a first embodiment of a supporting arm system 1 according to the present invention. Such supporting arm systems are especially used in the field of medical technology for the support of medical equipment or medical devices or for devices used in association with medical treatment rooms, such as medical lighting systems.

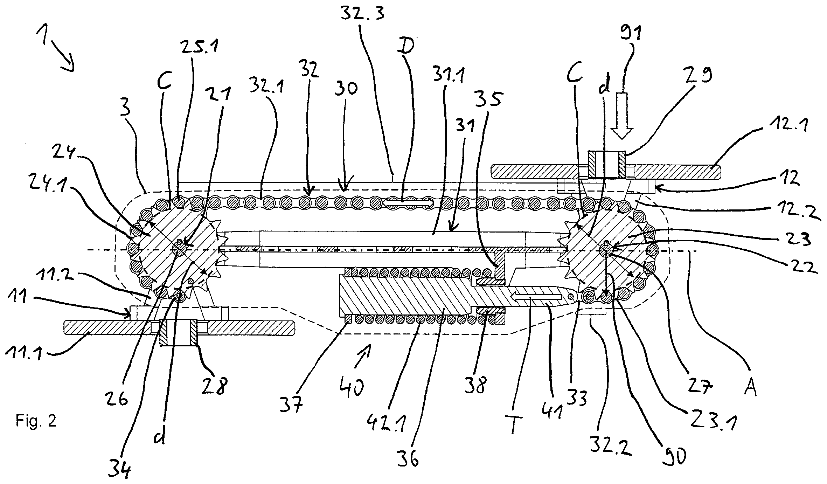

The supporting arm system 1 is arranged as a part of a movable stand 50 and is illustrated in a side view (in Figures 2-3 in a cut side view). The supporting arm system 1 has a supporting arm 30 which comprises a first supporting arm member 31 in the form of a load arm 31.1 and a second supporting arm member 32 in the form of a bendable tension intermediary 32.1. The bendable tension intermediary 32.1 is formed as a chain in this embodiment.

The supporting arm 30 is at one of its ends pivotably mounted at a first base element 11 by means of a first pivot bearing 26 in that its load arm 31.1 is mounted by means of the pivot bearing 26 at the first base element 11. The first base element 11 is formed in the present embodiment as a supporting element that comprises a base plate 11.1 and an axis support 11.2. The first base element 11 is mounted via its base plate 11.1 to the roller container 50.1 of the movable stand 50. The movable stand 50 may be a medical equipment or a part thereof. The first base element 11 and the roller container 50.1 are connected by means of a first swivel bearing 28, enabling a rotation of the supporting arm system 1 relative to the roller container 50.1. To temporarily disable rotation, there may be a breaking and/or locking mechanism located and being effective between the first base element 11 and the movable stand 50. On the first pivot axis 26 there is mounted a first curved guide member 24 in a non-rotatable manner. That means, while the load arm 31.1 may pivot around a first pivot axis 21 defined by the first pivot bearing 26, the first curved guide member 24 is static with respect to this pivot axis 21 and accordingly with respect to the first base element 11.

The supporting arm 30 is at its respective other end pivotably mounted at a second base element 12 by means of a second pivot bearing 27 in that its load arm 31.1 is mounted by means of the second pivot bearing 27 at the second base element 12. The second base element 12 is formed in the present embodiment as a load carrying element that comprises a base plate 12.1 and an axis support 12.2. Such load carrying element may carry or support medical devices, such as medical service heads with service outlets, lighting, one or more computer display screens, and/or any of a variety of medical or industrial equipment for use in a corresponding environment (e.g. hospital, medical practice, etc.).

In the present embodiment the second base element 12 is mounted via its base plate 12.1 to a carrier plate 12.3 on which a medical device 80 may be mounted, as indicated in Fig. 1. The medical device 80 may be a part of the medical equipment or movable stand 50. The second base element 12 and the carrier plate 12.3 are connected by means of a second swivel bearing 29, enabling a rotation of the carrier plate 12.3 with the medical device 80 relative to the supporting arm system 1. To temporarily disable rotation, there may be a breaking and/or locking mechanism located and being effective between the second base element 12 and the carrier plate 12.3. On the second pivot axis 27 there is mounted a second curved guide member 23 in a non-rotatable manner. This means, while the load arm 31.1 may pivot around a second pivot axis 22 defined by the second pivot bearing 27, the second curved guide member 23 is static with respect to this second pivot axis 22 and accordingly with respect to the second base element 12.

Preferably the first and second curved guide members 24, 23 have identical diameters d and circumferences C.

The bendable tension intermediary 32.1 is fixedly connected at one of its end portions 33 to a transmission member 41 of a tensioning means 40 and is further fixedly connected at its respective other end portion 34 to the first curved guide member 24. The bendable tension intermediary 32.1 is thereby guided around a part of the circumference C of the first curved guide member 24, and extends further to the second curved guide member 23. The bendable tension intermediary 32.1 is slip-free guided around a part of the circumference C of the second guide member 23. Slip-free guidance between the bendable tension intermediary 32.1 and the second curved guide member 23 is realized in this embodiment by a form fit between teeth 23.1 of the second curved guide member 23 which is formed as a gearwheel and the chain pins 25.1 of the bendable tension intermediary 32.1 which is formed as a chain.

Similarly, the bendable tension intermediary 32.1 is slip-free guided around a part of the circumference C of the first guide member 24. Slip-free guidance between the bendable tension intermediary 32.1 and the first curved guide member 24 is realized in this embodiment first by the fixed connection between the end portion 34 and the first curved guide member 24 and second by a form fit between teeth 24.1 of the first curved guide member 24 which is also formed as a gearwheel and the chain pins 25.1 of the bendable tension intermediary 32.1. With the slip-free guidance any slipping of the bendable tension intermediary 32.1 on the first or second curved guide members is prevented.

The bendable tension intermediary 32.1 is under a tension between the first and second curved guide members 23, 24 as indicated by double headed arrow D. The second curved guide member 23 defines a first section 32.2 of the bendable tension intermediary 32.1 extending between the tension means 40 and the second curved guide member 23. The second curved guide member 23 defines a second section 32.3 of the bendable tension intermediary 32.1 extending between the second curved guide member 23 and the first curved guide member 24. Both, the first section 32.2 and the second section 32.3 run in parallel to each other such that a slim outer contour of the supporting arm may be realized.

Tensioning means 40 comprise a compression spring 42.1 that is mounted at a mounting section 35 of the load arm 31.1 and is arranged such that a tension T of the spring 42.1 is effective in a direction parallel to a longitudinal axis A of the supporting arm 30 and the load arm 31.1. The longitudinal axis A of the load arm 31.1 intersects the first and second pivot axes 21, 22 and runs in parallel with the first section 32.2 and the second section 32.3 of the bendable tension intermediary 32.1.

The compression spring 42.1 is axially guided on a guide pillar 36 between a stop 37 and the mounting section 35. The guide pillar 36 is formed in one piece with the transmission member 41 which is formed as an extension of the guide pillar 36. The transmission member 41 has a smaller diameter than the guide pillar 36 and is axially slidably guided in a guide sleeve 38 of the mounting section 35.

To protect the supporting arm 30 it may be covered completely or partially with a housing 3, as indicated in Figures 1-3.

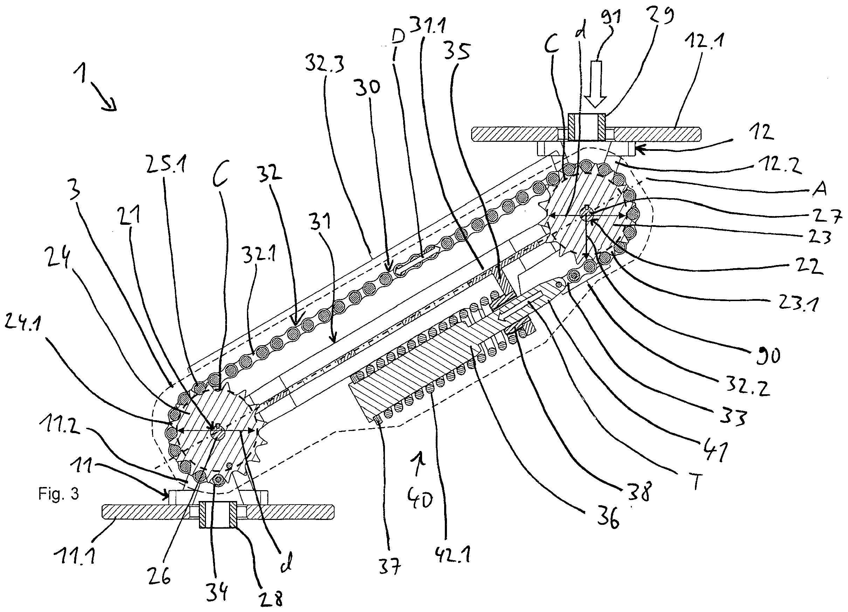

In Figures 1 and 2 the supporting arm system 1 is shown in its neutral position while Figure 3 displays a lifted position of the supporting arm 30 of the supporting arm system 1. In Figures 2 and 3 the arrow 90 indicates the rotary position of the second curved guide member 23 with respect to the second pivot axis 22 and the second base member 12 respectively. As can be seen from the similar position of arrow 90 in Figures 2 and 3, the rotary position of the second curved guide member 23 does not change when the load arm is moved upwards with the second base element 12. Similarly also the rotary position of the first curved guide member 24 does not change.

The function may be described as follows:

At the second base element 12 a load is applied (indicated by arrow 91). This load generates a momentum on the first pivot axis 21. This momentum is dependent from the height of the load F=m*g (with m= mass and g=gravitational constant) from the axial distance (a) of the first and second curved guide members 24, 23 along the longitudinal axis and from the pivot angle φ of the load arm. Accordingly the momentum ($M_L$) is given by:

$$M_L(\varphi) = F * a * \cos(\varphi) = m * g * a * \cos(\varphi)$$

The tensioning means 40 / pressure spring 42.1 generates a force which is redirected by the bendable tension member 32.1 / the chain around the second curved guide member 23 and effects on the circumference C of the first curved guide member 24.

According to the invention, the counter momentum caused by the tensioning means 40 / pressure spring 42.1 is approximated to the momentum ($M_L$) of the load such that the supporting arm 30 remains in a stable equilibrium.

Counter momentum ($M_S$) of the tensioning means 40 / pressure spring 42.1 is accordingly given by:

$$M_S(\varphi) = s_v * R * r - \varphi * \frac{\pi}{180^\circ} * R * r^2$$

with

    φ: pivot angle of the load arm 31.1 in degrees (Fig. 1 and 2 display neutral (0°) position, Fig. 3 displays 30° position),
    $s_v$: pre-tension of the spring 42.1 in the neutral (0°) position as indicated in Fig. 1 and 2,
    R: spring constant,
    r: effective radius of the first curved guide member 24 (in this embodiment equal to the radius of the second curved guide member 23).

During an upward motion of the supporting arm 30 the bendable tension intermediary 32.1 / the chain unrolls on the first curved guide member 24. Since the bendable tension intermediary 32.1 / chain is kept under tension by the tensioning means 40 / the pressure spring 42.1, the freed length is unrolled over the second curved guide member 24, while the tensioning means 40 / the pressure spring 42.1 relaxes and the transmission member moves further inside the pressure spring 42.1. A downward movement leads accordingly to a rolling up of the bendable tension intermediary 32.1 / the chain on the first curved guide member 24 and while stretching the tensioning means 40 / the pressure spring 42.1. The length, over which the feather becomes stretched during the movement, corresponds to the arc length (b) of the rolled on and/or rolled up bendable tension intermediary 32.1 / chain and can be described by:

$$b = \varphi * \frac{\pi}{180^\circ} * r.$$

An increase of the load capacity of the supporting arm system can easily be obtained by increasing the pre-tension of the tensioning means 40 / pressure spring 42.1. If much higher or smaller load capacitances are required, either first and second curved guide members with a larger or smaller diameter may be used or a tensioning means 40 comprising a spring with a different (smaller or larger) spring constant may be chosen.

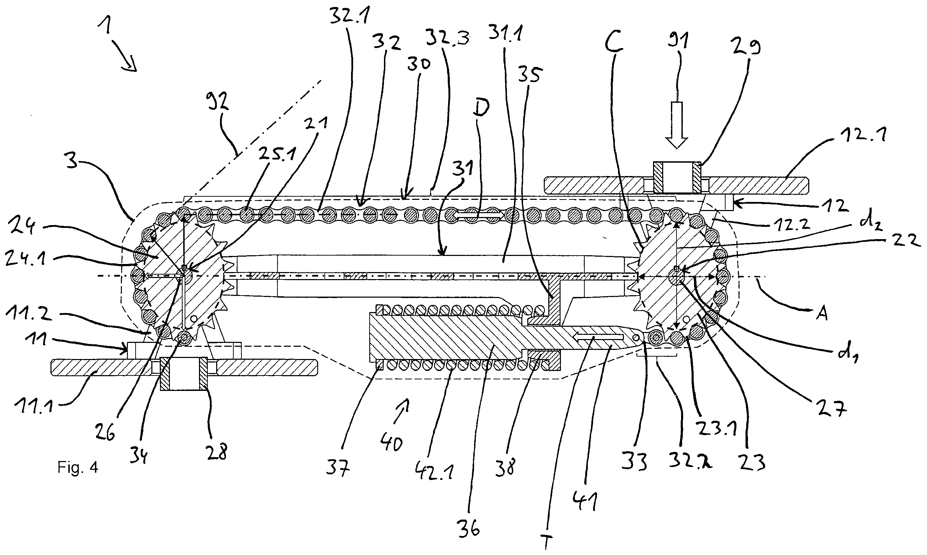

Figure 4 displays a further embodiment of the supporting arm system 1. For reference numerals and functions not described in the following text to this embodiment, reference is made to the description of Figs. 1 to 3 which is herewith incorporated by reference.

The embodiment of Fig. 4 differs from the one described in Figs. 1 to 3 in that both, the first and second curved guide members 24, 23 are formed as elliptical gearwheels. Each of the gearwheels have a first smaller diameter $d_1$ and a second larger diameter $d_2$, wherein the first smaller diameter $d_1$ and the second larger diameter $d_2$ are perpendicular to each other. With such elliptical configuration of the gearwheels the non-linear momentum ($M_L$) at the first pivot axis can be approximated further to the counter momentum of the tensioning means 40 / pressure spring 42.1 such that the equilibrium of the supporting arm 30 is optimized. The position of the bendable tension intermediary 32.1 / chain in a lifted position of the supporting arm 30 (not shown) is indicated in Figure 4 by the dot-dashed line 92 which indicates the unrolling of the bendable tension intermediary 32.1 / chain from the first curved guide member 24.

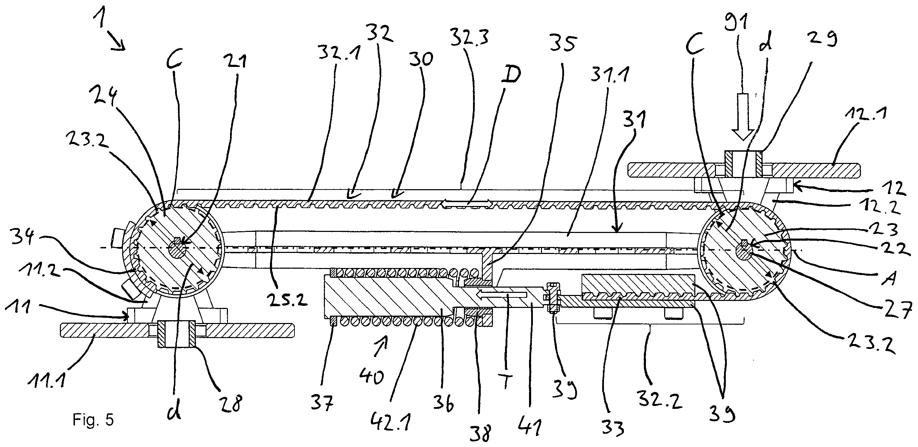

Figure 5 displays a further embodiment of the supporting arm system 1. For reference numerals and functions not described in the following text to this embodiment, reference is made to the description of Figs. 1 to 3 which is herewith incorporated by reference.

The embodiment of Fig. 5 differs from the one described in Figs. 1 to 3 in that the bendable tension intermediary 32.1 is formed as a belt and in that the first and second curved guide members 24, 23 are formed as belt wheels which carry teeth 23.2 on their circumference C which engage with corresponding belt teeth 25.2 of the belt. The function of the belt and belt wheel combination is similar to the one described before with reference to Figures 1 to 3. It may though be mentioned, that the belt wheels may also be elliptical with a first smaller diameter $d_1$ and a second larger diameter $d_2$, wherein the first smaller diameter $d_1$ and the second larger diameter $d_2$ are perpendicular to each other. The belt is coupled to the transmission member by means of a coupling 39 consisting at least of a clamp plate and connector members such as one or more screws.

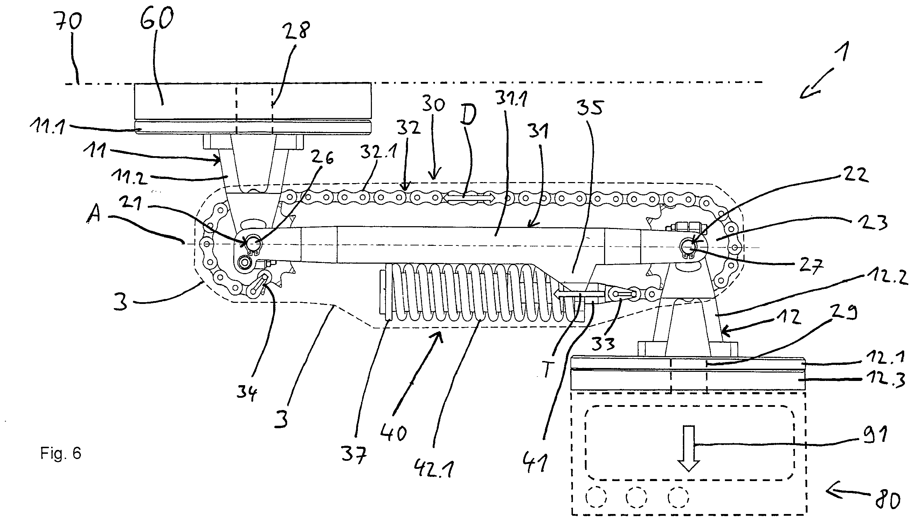

Figure 6 displays a further embodiment of the supporting arm system 1. For reference numerals and functions not described in the following text to this embodiment, reference is made to the description of Figs. 1 to 3 which is herewith incorporated by reference.

The embodiment of Fig. 6 differs from the one described in Figs. 1 to 3 in that the first base element 11 is connected to a ceiling mount 60 which is mounted at a ceiling 70. Between the ceiling mount 60 and the base plate 11.1 of the first base element 11 there is a first swivel bearing 28 by means of which the supporting arm system 1 is rotatable relative to the ceiling mount 60. To temporarily disable rotation, there may be a breaking and/or locking mechanism located and being effective between the first base element 11 and the ceiling mount 60.

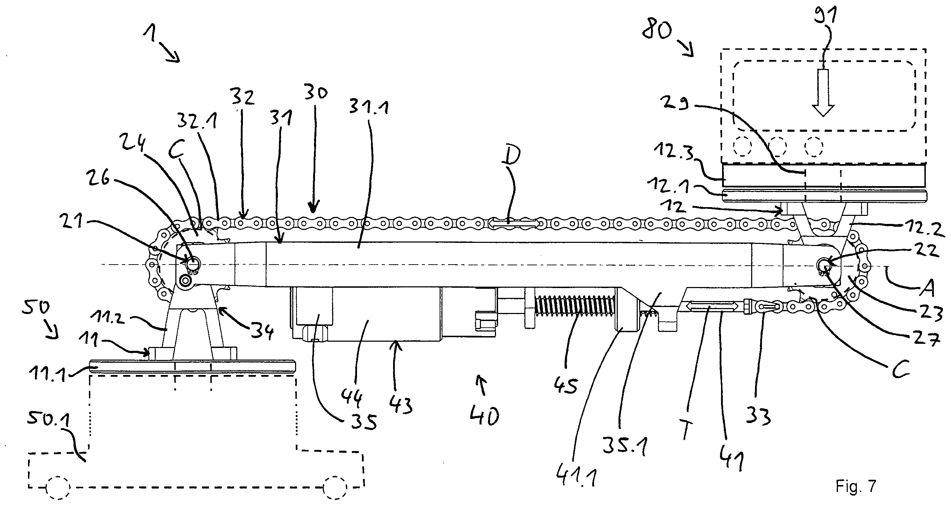

Figure 7 displays a further embodiment of the supporting arm system 1. For reference numerals and functions not described in the following text to this embodiment, reference, is made to the description of Figs. 1 to 3 which is herewith incorporated by reference.

The embodiment of Figure 7 differs from the one described in Figures 1 to 3 in that the tensioning means 40 comprises a motorized actuator 43 which includes a motor 44 (preferably an electrically driven motor) which is connected to a worm gear 45. The motorized actuator 43 is used instead of the spring elements used in the embodiments described before and exerts the tension T on the bendable tension intermediary 32.1 when actuated in the tension direction. The motor 44 is mounted at the load arm 31.1 by means of a mounting section 35. The worm gear 45 is rotatably mounted in a mounting and guiding section 35.1 which is formed as an extension of the load arm 31.1. The transmission member 41 includes a slider member 41.1 which has an internal thread and combs the worm gear 45 such that a rotational movement of the worm gear 45 is converted into an axial movement of the transmission member 41. If the supporting arm system is in a lifted position a downward movement of the supporting arm 30 with a load 91 is hindered by the self-locking effect of the worm gear.

The motorized actuator 43 may be controlled by a control unit (not shown) through which a person may control lifting and lowering of the supporting arm system 1. The control unit is connected to the motorized actuator 43 either wireless or by cable.

With the motorized actuator 43 it is possible to automatically raise or lower the supporting arm 30 with the second base element 12 and a medical device 80 being arranged on the carrier plate 12.3 of the second base element. Alternatively to a motor 44 other types of electrically controllable actuators may be used such as servos or solenoids.

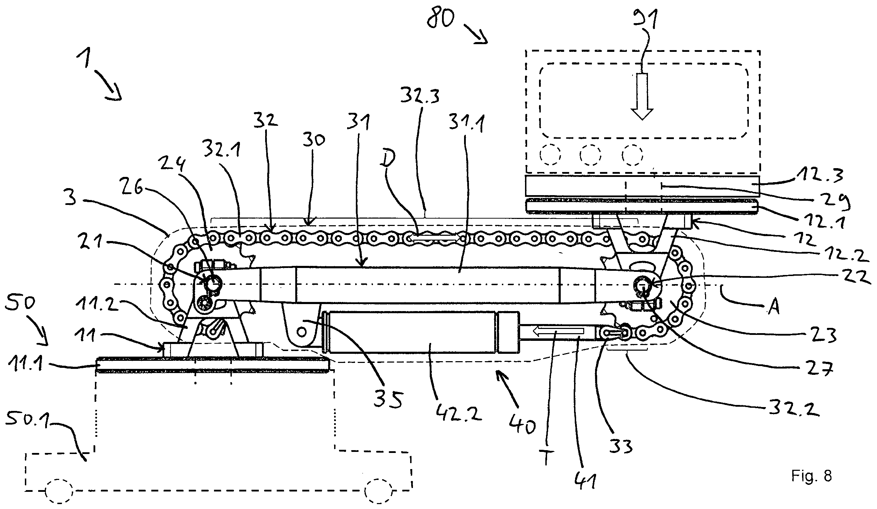

Figure 8 displays a further embodiment of the supporting arm system 1. For reference numerals and functions not described in the following text to this embodiment, reference is made to the description of Figs. 1 to 3 which is herewith incorporated by reference.

The embodiment of Fig. 8 differs from the one described in Figs. 1 to 3 in that the tensioning means 40 comprise a gas spring 42.2 instead of a pressure or spiral spring as has been described with respect to Figures 1-3.

It will be appreciated that the above description of the preferred embodiments of the invention with reference to the drawings has been made by way of example only. Thus, a person skilled in the art will appreciate that various changes, modifications and/or additions may be made to the parts particularly described and illustrated without departing from the scope of the invention as defined in the claims.

REFERENCE LIST

1       supporting arm system
3       housing
11      first base element

| | |
|---|---|
| 11.1 | base plate |
| 11.2 | axis support |
| 12 | second base element |
| 12.1 | base plate |
| 12.2 | axis support[SN1] |
| 12.3 | carrier plate |
| | |
| 21 | first pivot axis |
| 22 | second pivot axis |
| 23 | second curved guide member |
| 23.1 | teeth |
| 23.2 | teeth |
| 24 | first curved guide member |
| 24.1 | teeth |
| 25.1 | chain pin |
| 25.2 | belt tooth |
| 26 | first pivot bearing |
| 27 | second pivot bearing |
| 28 | first swivel bearing |
| 29 | second swivel bearing |
| 30 | supporting arm |
| 31 | first supporting arm member |
| 31.1 | load arm |
| 32 | second supporting arm member |
| 32.1 | bendable tension intermediary |
| 32.2 | first section (of the bendable tension intermediary) |
| 32.3 | second section (of the bendable tension intermediary) |
| 33 | end portion (of the bendable tension intermediary) |
| 34 | other end portion (of the bendable tension intermediary) |
| 35 | mounting section |
| 35.1 | mounting and guiding section |
| 36 | guide pillar |
| 37 | stop |
| 38 | guide sleeve |
| 39 | coupling |
| 40 | tensioning means |
| 41 | transmission member |
| 42.1 | pressure spring |
| 42.2 | gas spring |
| 43 | motorized actuator |
| 44 | motor |
| 45 | worm gear |
| | |
| 50 | element, movable stand |
| 50.1 | roller container |
| | |
| 60 | element, ceiling mount |
| 70 | ceiling |
| 80 | medical device |
| 90 | arrow |
| 91 | arrow (load) |
| 92 | dot-dashed line |
| A | longitudinal axis |
| C | circumference |
| D | direction of tension |
| d | diameter |
| $d_1$ | diameter |
| $d_2$ | diameter |

T      tension force

**Claims**

1. Supporting arm system (1) for supporting technical equipment, especially medical equipment, comprising:

   a first base element (11) configured to be connected to a further element,
   a second base element (12) for carrying a technical device, especially a medical device (80),
   a supporting arm (30) pivotally mounted to the first base element (11) at a first pivot axis (21) and pivotally mounted to the second base element (12) at a second pivot axis (22); the supporting arm (30) comprising a first supporting arm member (31) formed as a load arm (31.1) and a second supporting arm member (32) for stabilizing a position of the load arm,
   and tensioning means (40) connected to the second supporting arm member (31) for exerting a tension force (T) on the second supporting arm member (32),
   **characterized in that**
   the second supporting arm member (32) is formed as a bendable tension intermediary (32.1) which is fixedly connected at one end portion (33) to a transmission member (41) of the tensioning means (40) and which is fixedly connected at its respective other end portion (34) to a first curved guide member (24) that is non-rotatably mounted on the first pivot axis (21) with the bendable tension intermediary (32.1) being guided around a part of the circumference (C) of the first curved guide member (24), and
   wherein a second curved guide member (23) is non-rotatably mounted on the second pivot axis (22) with the bendable tension intermediary (32.1) being slip-free guided around a part of the circumference (C) of the second guide member (23).

2. Supporting arm system (1) according to claim 1, **characterized in that** the tensioning means (40) is fixedly mounted at the load arm (31.1).

3. Supporting arm system (1) according to claim 1 or 2, **characterized in that**
   the second curved guide member (23) defines a first section (32.2) of the bendable tension intermediary (32.1) extending between the tension means (40) and the second curved guide member (23), and
   the second curved guide member (23) defines a second section (32.3) of the bendable tension intermediary (32.1) extending between the second curved guide member (23) and the first curved guide member (24), and
   wherein the first section (32.2) and the second section (32.3) run in parallel to each other.

4. Supporting arm system (1) according to claim 3, **characterized in that** a longitudinal axis (A) of the load arm (31.1) intersects the first and second pivot axes (21, 22) and runs in parallel with the first section (32.2) and the second section (32.3) of the bendable tension intermediary (32.1).

5. Supporting arm system (1) according to any one of the preceding claims, **characterized in that** the bendable tension intermediary (32.1) is formed as one of a chain, belt, rope or wire rope.

6. Supporting arm system (1) according to any one of the preceding claims, **characterized in that** the first and second curved guide members (24, 23) are formed as one of gearwheels, belt wheels or pulleys.

7. Supporting arm system (1) according to any one of the preceding claims, **characterized in that** the tensioning means (40) comprises at least one spring element from the group consisting of tension spring, pressure spring (42.1) and gas spring (42.2).

8. Supporting arm system (1) according to any one of claims 1 to 6, **characterized in that** the tensioning means (40) comprises a motorized actuator (43).

9. Supporting arm system (1) according to claim 8, **characterized in that** the motorized actuator (43) includes an electrically controllable motor (44) which is coupled to the transmission member (41) that is connected to the bendable tension intermediary (32.1).

10. Supporting arm system (1) according to claim 9, **characterized in that** the motor (44) cooperates with a worm gear (45) that is coupled to the transmission member (41).

**11.** Supporting arm system (1) according to any of the preceding claims, **characterized in that** the first and second curved guide members (24, 23) have identical diameters (d, $d_1$, $d_2$) and circumferences (C).

**12.** Supporting arm system (1) according to any one of the preceding claims, **characterized in that** the first and second curved guide members (24, 23) are elliptical having a first smaller diameter ($d_1$) and a second larger diameter ($d_2$), wherein the first smaller diameter ($d_1$) and the second larger diameter ($d_2$) are perpendicular to each other.

**13.** A stand (50) for supporting technical equipment, especially medical equipment in a hospital or other medical environment, comprising one or more supporting arm systems (1) according to one or more of the preceding claims.

**14.** A ceiling mount (60) for supporting technical equipment, especially medical equipment in a hospital or other medical environment, comprising one or more supporting arm systems (1) according to one or more of the preceding claims 1 to 12.

## Patentansprüche

**1.** Tragarmsystem (1) zum Tragen von technischen Geräten, insbesondere medizinischen Geräten, umfassend:

ein erstes Basiselement (11), das konfiguriert ist, um mit einem weiteren Element verbunden zu werden,
ein zweites Basiselement (12) zum Tragen einer technischen Vorrichtung, insbesondere einer medizinischen Vorrichtung (80),
einen Tragarm (30), der schwenkbar an dem ersten Basiselement (11) an einer ersten Schwenkachse (21) und schwenkbar an dem zweiten Basiselement (12) an einer zweiten Schwenkachse (22) befestigt ist; wobei der Tragarm (30) ein erstes Tragarmteil (31), das als Lastarm (31.1) ausgebildet ist, und ein zweites Tragarmteil (32) zum Stabilisieren einer Position des Lastarms umfasst,
und Spannmittel (40), die mit dem zweiten Tragarmteil (31) verbunden sind, um eine Spannkraft (T) auf das zweite Tragarmteil (32) auszuüben,
**dadurch gekennzeichnet, dass**
das zweite Tragarmteil (32) als biegsames Spannungszwischenteil (32.1) ausgebildet ist, das an einem Endabschnitt (33) fest mit einem Übertragungselement (41) des Spannmittels (40) verbunden ist und das an seinem anderen Endabschnitt (34) fest mit einem ersten gekrümmten Führungselement (24) verbunden ist, das an der ersten Drehachse (21) drehfest gelagert ist, wobei das biegsame Spannungszwischenteil (32.1) um einen Teil des Umfangs (C) des ersten gekrümmten Führungselements (24) geführt ist, und
wobei ein zweites gekrümmtes Führungselement (23) drehfest auf der zweiten Schwenkachse (22) montiert ist, wobei das biegsame Spannungszwischenteil (32.1) schlupffrei um einen Teil des Umfangs (C) des zweiten Führungselements (23) geführt ist.

**2.** Tragarmsystem (1) nach Anspruch 1, **dadurch gekennzeichnet, dass** das Spannmittel (40) fest am Lastarm (31.1) gelagert ist.

**3.** Tragarmsystem (1) nach Anspruch 1 oder 2, **dadurch gekennzeichnet, dass**
das zweite gekrümmte Führungselement (23) einen ersten Abschnitt (32.2) des biegsamen Spannungszwischenteils (32.1) definiert, das sich zwischen dem Spannmittel (40) und dem zweiten gekrümmten Führungselement (23) erstreckt, und
das zweite gekrümmte Führungselement (23) einen zweiten Abschnitt (32.3) des biegsamen Spannungszwischenteils (32.1) definiert, der sich zwischen dem zweiten gekrümmten Führungselement (23) und dem ersten gekrümmten Führungselement (24) erstreckt, und
wobei der erste Abschnitt (32.2) und der zweite Abschnitt (32.3) parallel zueinander verlaufen.

**4.** Tragarmsystem (1) nach Anspruch 3, **dadurch gekennzeichnet, dass** eine Längsachse (A) des Lastarms (31.1) die erste und zweite Drehachse (21, 22) schneidet und parallel zum ersten Abschnitt (32.2) und zum zweiten Abschnitt (32.3) des biegsamen Spannungszwischenteils (32.1) verläuft.

**5.** Tragarmsystem (1) nach einem der vorstehenden Ansprüche, **dadurch gekennzeichnet, dass** das biegsame Spannungszwischenteil (32.1) als Kette, Riemen, Seil oder Drahtseil ausgebildet ist.

**6.** Tragarmsystem (1) nach einem der vorstehenden Ansprüche, **dadurch gekennzeichnet, dass** das erste und zweite gekrümmte Führungselement (24, 23) als Zahnräder, Riemenscheiben oder Umlenkrollen ausgebildet sind.

7. Tragarmsystem (1) nach einem der vorstehenden Ansprüche, **dadurch gekennzeichnet, dass** das Spannmittel (40) mindestens ein Federelement aus der Gruppe bestehend aus Zugfeder, Druckfeder (42.1) und Gasfeder (42.2) umfasst.

8. Tragarmsystem (1) nach einem der Ansprüche 1 bis 6, **dadurch gekennzeichnet, dass** das Spannmittel (40) ein motorisiertes Stellglied (43) umfasst.

9. Tragarmsystem (1) nach Anspruch 8, **dadurch gekennzeichnet, dass** das motorisierte Stellglied (43) einen elektrisch steuerbaren Motor (44) beinhaltet, der mit dem Übertragungselement (41) gekoppelt ist, das mit dem biegsamen Spannungszwischenteil (32.1) verbunden ist.

10. Tragarmsystem (1) nach Anspruch 9, **dadurch gekennzeichnet, dass** der Motor (44) mit einem Schneckengetriebe (45) zusammenwirkt, das mit dem Übertragungselement (41) gekoppelt ist.

11. Tragarmsystem (1) nach einem der vorstehenden Ansprüche, **dadurch gekennzeichnet, dass** das erste und zweite gekrümmte Führungselement (24, 23) identische Durchmesser ($d$, $d_1$, $d_2$) und Umfänge ($C$) aufweisen.

12. Tragarmsystem (1) nach einem der vorhergehenden Ansprüche, **dadurch gekennzeichnet, dass** das erste und zweite gekrümmte Führungselement (24, 23) elliptisch mit einem ersten kleineren Durchmesser ($d_1$) und einem zweiten größeren Durchmesser ($d_2$) sind, wobei der erste kleinere Durchmesser ($d_1$) und der zweite größere Durchmesser ($d_2$) senkrecht zueinander stehen.

13. Ständer (50) zum Tragen von technischer Ausrüstung, insbesondere medizinischer Ausrüstung in einem Krankenhaus oder einer anderen medizinischen Umgebung, umfassend ein oder mehrere Tragarmsysteme (1) gemäß einem oder mehreren der vorstehenden Ansprüche.

14. Deckenhalterung (60) zum Tragen von technischen Geräten, insbesondere medizinischen Geräten in einem Krankenhaus oder einer anderen medizinischen Umgebung, umfassend ein oder mehrere Tragarmsysteme (1) gemäß einem oder mehreren der vorstehenden Ansprüche 1 bis 12.

**Revendications**

1. Système de bras de support (1) pour supporter un équipement technique, en particulier un équipement médical, comprenant :

un premier élément de base (11) configuré pour être connecté à un autre élément,
un deuxième élément de base (12) pour porter un dispositif technique, en particulier un dispositif médical (80),
un bras de support (30) monté pivotant sur le premier élément de base (11) au niveau d'un premier axe de pivotement (21) et monté pivotant sur le deuxième élément de base (12) au niveau d'un deuxième axe de pivotement (22) ; le bras de support (30) comprenant un premier élément de bras de support (31) formé comme bras de charge (31.1) et un deuxième élément de bras de support (32) pour stabiliser une position du bras de charge,
et des moyens de tension (40) reliés au deuxième élément de bras de support (31) pour exercer une force de tension (T) sur le deuxième élément de bras de support (32),
**caractérisé en ce que**
le deuxième élément de bras de support (32) est réalisé sous la forme d'un intermédiaire de tension pliable (32.1) qui est relié de manière fixe à une partie d'extrémité (33) à un élément de transmission (41) du moyen de tension (40) et qui est relié de manière fixe à son autre partie d'extrémité respective (34) à un premier élément de guidage courbe (24) qui est monté non rotatif sur le premier axe de pivotement (21), l'intermédiaire de tension flexible (32.1) étant guidé sur une partie du périmètre (C) du premier élément de guidage courbe (24), et dans laquelle un deuxième élément de guidage incurvé (23) est monté de manière non rotative sur le deuxième axe de pivotement (22), l'intermédiaire de tension pliable (32.1) étant guidé sans glissement sur une partie de la périphérie (C) du deuxième élément de guidage (23).

2. Système de bras porteur (1) selon la revendication 1, **caractérisé en ce que** le moyen de tension (40) est monté de manière fixe sur le bras porteur (31.1).

3. Système de bras de support (1) selon la revendication 1 ou 2, **caractérisé en ce que** le deuxième élément de guidage courbe (23) définit une première section (32.2) de l'intermédiaire de tension pliable (32.1) s'étendant entre le moyen de tension (40) et le deuxième élément de guidage courbe (23), et
le second élément de guidage incurvé (23) définit une seconde section (32.3) de l'intermédiaire de tension pliable (32.1) s'étendant entre le second élément de guidage incurvé (23) et le premier élément de guidage incurvé (24), et dans laquelle la première section (32.2) et la deuxième section (32.3) sont parallèles l'une à l'autre.

4. Système de bras porteur (1) selon la revendication 3, **caractérisé en ce qu'**un axe longitudinal (A) du bras porteur (31.1) croise les premier et second axes de pivotement (21, 22) et s'étend parallèlement à la première section (32.2) et à la seconde section (32.3) de l'intermédiaire (32.1) de tension pliable.

5. Système de bras porteur (1) selon l'une quelconque des revendications précédentes, **caractérisé en ce que** l'intermédiaire de tension pliable (32.1) est réalisé sous la forme d'une chaîne, d'une courroie, d'un câble ou d'un câble métallique.

6. Système de bras porteur (1) selon l'une quelconque des revendications précédentes, **caractérisé en ce que** les premier et second éléments de guidage courbés (24, 23) sont réalisés sous la forme d'une roue dentée, d'une roue de courroie ou de poulies.

7. Système de bras porteur (1) selon l'une quelconque des revendications précédentes, **caractérisé en ce que** le moyen de tension (40) comprend au moins un élément de ressort du groupe constitué par un ressort de tension, un ressort de pression (42.1) et un ressort pneumatique (42.2).

8. Système de bras de support (1) selon l'une quelconque des revendications 1 à 6, **caractérisé en ce que** le moyen de tension (40) comprend un actionneur motorisé (43).

9. Système de bras porteur (1) selon la revendication 8, **caractérisé en ce que** l'actionneur motorisé (43) comprend un moteur (44) pouvant être commandé électriquement qui est couplé à l'élément de transmission (41) qui est relié à l'intermédiaire de tension pliable (32.1).

10. Système de bras porteur (1) selon la revendication 9, **caractérisé en ce que** le moteur (44) coopère avec un engrenage à vis sans fin (45) qui est couplé à l'organe de transmission (41).

11. Système de bras porteur (1) selon l'une quelconque des revendications précédentes, **caractérisé en ce que** les premier et second éléments de guidage courbés (24, 23) ont des diamètres (d, d1, d2) et des circonférences (C) identiques.

12. Système de bras de support (1) selon l'une quelconque des revendications précédentes, **caractérisé en ce que** les premier et second éléments de guidage incurvés (24, 23) sont elliptiques ayant un premier diamètre inférieur (d1) et un second diamètre supérieur (d2), dans lequel le premier diamètre inférieur (d1) et le second diamètre supérieur (d2) sont perpendiculaires entre eux.

13. Support (50) pour supporter un équipement technique, en particulier un équipement médical dans un hôpital ou un autre environnement médical, comprenant un ou plusieurs systèmes de bras de support (1) selon une ou plusieurs des revendications précédentes.

14. Support de plafond (60) pour supporter un équipement technique, en particulier un équipement médical dans un hôpital ou un autre environnement médical, comprenant un ou plusieurs systèmes de bras de support (1) selon une ou plusieurs des revendications précédentes 1-12.

Fig. 1

Fig. 2

Fig. 3

Fig. 4

EP 3 275 394 B1

16

Fig. 5

EP 3 275 394 B1

Fig. 6

Fig. 7

Fig. 8

**EP 3 275 394 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080111042 A1
- DE 3312137 A1
- US 20070187562 A1